# EUROPÄISCHE PATENTANMELDUNG

(11) **EP 1 693 071 A2**
(43) Veröffentlichungstag der Anmeldung: **23.08.2006**
(21) Anmeldenummer: 06002522.8
(22) Anmeldetag: 08.02.2006
(51) Int. Cl.: A61K 47/48, A61K 8/64, A61Q 19/00

(54) **Kovalent gebundene Wirkkomplexe, aus denen Stress aktivierbare Hautenzyme einen Wirkstoff freisetzen**

(30) Priorität: 16.02.2005 DE 102005007468
(71) Anmelder: Beiersdorf Aktiengesellschaft, D-20253 Hamburg (DE)
(72) Erfinder: Stäb, Franz, Dr., 21379 Echem (DE); Blatt, Thomas, Dr., 22880 Wedel (DE); Gallinat, Stefan, Dr., 22880 Wedel (DE); Breitenbach, Ute, Dr., 20251 Hamburg (DE); Scherner, Cathrin, Dr., 22455 Hamburg (DE)

(57) **Zusammenfassung**

Wirkkomplex enthaltend die Substrukturen A und B, wobei jeweils mindestens ein A und B durch mindestens eine kovalente Bindung verbunden sind und A ein durch Stress aktivierbare Hautenzyme abspaltbares Substratfragment ist und B ein kosmetischer und/oder dermatologischer Wirkstoff.

## Beschreibung

Die vorliegende Erfindung betrifft neuartige Wirkkomplexe aus kovalent mit durch Stress aktivierbaren Hautenzymen abspaltbaren Substratfragmenten verbundenen kosmetischen und/oder dermatologischen Wirkstoffen.

Substratfragmente im Sinne der vorliegenden Erfindung sind biologische und chemische Strukturen, die an Wirkstoffe gekoppelt vorliegen, und zusätzlich für bestimmte Enzyme typische Spaltstellen aufweisen. In diesem Sinne können sie als Substrat des jeweiligen Enzyms angesehen werden, das auf erfindungsgemäß beschriebene Weise aktiviert werden kann. Da die nach Enzymaktivität verbleibenden Spaltprodukte jeweils einzeln kosmetisch wirksam sind, kann man auch von einem aktivierbaren Wirkkomplex sprechen.

Der Begriff umfasst auch als Linker bezeichnete Strukturen. Dabei handelt es sich um verkürzte Substrate, die sich vom physiologischen Target ableiten lassen und nach Stress durch die jeweiligen Enzym gespalten werden und die beiden Einzeiwirkstoffe freisetzen.

Pigmentierungsmodulatoren im Sinne der vorliegenden Erfindung sind diejenigen biologischen und chemischen Strukturen, die nach erfindungsgemäß erfolgter Enzymaktivität als verbleibende Spaltprodukte das Bräunungs- und Pigmentierungsverhalten der Haut modulieren können, indem sie auf beteiligten Zelltypen einwirken können.

Anti-entzündliche Wirkstoffe im Sinne der vorliegenden Erfindung sind diejenigen biologischen und chemischen Strukturen, die nach erfindungsgemäß erfolgter Enzymaktivität als verbleibende Spaltprodukte antientzündliche Vorgänge in der Haut modulieren können, indem sie auf beteiligten Zelltypen einwirken können.

Hautbefeuchter im Sinne der vorliegenden Erfindung sind diejenigen biologischen und chemischen Strukturen, die nach erfindungsgemäß erfolgter Enzymaktivität als verbleibende Spaltprodukte Vorgänge in der Haut modulieren können, indem sie die Regulation der hauteigenen Natural Moisturizer, Wasserbindungskapazität positiv beeinflussen und/oder den kutanen Wasserverlust reduzieren können.

Energisator im Sinne der vorliegenden Erfindung sind Substanzen, die den Energiestoffwechsel von Zellen, insbesondere der Haut und Hautanhangsgebilde, positiv beeinflussen, wie z.B. Kreatin und/oder Kreatinin, Q10 und Carnitin. Sie werden auch als "energizer" bezeichnet.

Durch Stress aktivierbare Hautenzyme im Sinne der vorliegenden Erfindung sind bindegewebs-, bevorzugt kollagenabbauende, hyaluronanabbauende und eiastinabbauende Enzyme wie Endopeptidasen, Matrixmetalloproteinasen, Elastasen und Hyaluronidasen.

Kollagenabbauende Endopeptidasen (Kollagehasen) sind Enzyme, die die Strukturproteine des Bindegewebes degradieren und für den Abbau von Elastin- und Kollagenfasern, aber auch von Proteoglykanen verantwortlich sind. Die kontrollierte Aktivität dieser Enzyme spielt eine entscheidende Rolle für die Gewebeumstrukturierung während Prozessen der Entwicklung, der Gewebereparatur sowie der Angiogenese.

### Stand der Technik

Grundsätzlich besteht bei kosmetischen Zubereitungen das Problem, dass sie großflächig appliziert werden und damit wertvolle Inhaltstoffe, die Zellen oder Zellareale positiv beeinflussen, quasi mit der Gießkanne verteilt werden, ohne genau an der Stelle angewendet zu werden, an der ihr Einsatz eigentlich erwünscht ist. Wünschenswert wäre es, kosmetische oder dermatologische Wirkstoffe nur dort wirken zu lassen, wo dies auch gewünscht ist. Beispiele wären Einsatz hautberuhigender Wirkstoffe allein an gestressten Hautpartien oder der Einsatz Kollagenasen hemmender Wirkstoffe gezielt an Hautpartien mit verstärkten Kollagenabbau. Der Anwender kann jedoch nicht selbst feststellen, wo genau ein bestimmter Wirkstoff appliziert werden müsste, weil die Notwendigkeit des Einsatzes für den Menschen nicht wahrnehmbar ist. Muss der Einsatz von Wirkstoffen in tieferen Hautschichten erfolgen, so sind diese darüber hinaus für einen topisch applizierten Wirkstoff nicht direkt erreichbar. Auch sehr kleine Applikationsarreale, beispielsweise wenige betroffene Zellen, sind einer gezielten kosmetischen Behandlung zur Zeit nicht zugänglich.

WO 02/13820 offenbart topische Zubereitungen für die photodynamische Therapie von Gewebeerkrankungen, deren Inhaltstoffe durch gerichtete Strahlung wie Laser aktiviert werden und so an vorher durch den Behandelnden ausgewählten Hautarealen wirken.

Es wurde nun überraschend und für den Fachmann nicht vorhersehbar gefunden, daß die Nachteile des Standes der Technik überwunden werden durch einen Wirkkomplex enthaltend die Substrukturen A und B, wobei jeweils mindestens ein A und B durch mindestens eine kovalente Bindung verbunden sind und A ein durch Stress aktivierbare Hautenzyme abspaltbares Substratfragment ist, bevorzugt ein Oligopeptid mit mindestens 2 Aminosäuren und B ein kosmetischer und/oder dermatölogischer Wirkstoff.

Dieser Wirkkomplex weist folgende Vorteile auf:
- die Aktivierung des Wirkstoffkomplexes erfolgt zu dem Zeitpunkt, wenn die Aktivität der schädigenden Enzyme ansteigt und ihr Maximum erreicht, wenn also der Bedarf am größten ist. Die Aktivierung des Prodrugs (also des inaktiven Wirkkomplexes) erfolgt quasi ohne zeitliche Verzögerung.
- Der jeweilige Wirkstoff kann - je nach chemischer Struktur - N- bzw. C-terminal an den verwendeten Linker gekoppelt werden.
- Die Linker leiten sich dabei von den unten genannten Sequenzen ab, können aber auch um einige Aminosäuren verkürzt werden.

Es wurde weiter gefunden, daß die kovalente Bindung bevorzugt eine Ester-, Acetal-, Peptid- oder Amidbindung darstellt. Bevorzugt ist das durch Stress aktivierbare Hautenzym eine Elastase, Hyaluronidase oder Metalloproteinase. Besonders bevorzugt ist es, wenn als Wirkstoff B ein Hydroxyl-, Carboxyl-, Keto- oder Aminogruppen tragendes Antifalten- oder Lichtschutzmittel, Antioxidans, ein Pigmentierungsmodulator (Tanning, whitening, Altersflecken), ein anti-entzündlicher Wirkstoff, ein Hautbefeuchter oder ein Energisator gewählt wird. Ganz besonders bevorzugt ist es, wenn als Hydroxylgruppen tragender Wirkstoff B ein Isoflavonoid, bevorzugt Genistein, Sylimarin, Daidzein, Arctiin, Arctigenin, Ubiquinol (reduziertes Q10), Mevalonsäure, ein Vitamin, besonders bevorzugt Vitamin A, C oder E, Cholesterin und/oder Licochalcon A gewählt wird. Dabei ist es insbesondere bevorzugt, wenn als Carboxylgruppen tragender Wirkstoff B Kreatin, Mevalonsäure und Vitamin C gewählt wird, als Ketogruppen tragender Wirkstoff B Isoflavonoide, insbesondere Genistein, Arctiin, Arctigenin, Q10, Peptidische Wirkstoffe (insbesondere Tri- bis Decapeptide), Licochalcone A, Liponamid und/oder Taurin gewählt wird, als Aminogruppen tragender Wirkstoff B Kreatin, ein peptidischer Wirkstoff, besonders bevorzugt Tri- bis Decapeptide, Liponamid und/oder Taurin gewählt wird, als Substratfragment A ein Hydroxyl-, Carboxyl-, Keto- oder Aminogruppen tragendes Substratfragment gewählt wird, als Substratfragment A eines der Oligopeptide Pro-Leu-Ala-Leu-Trp-Ala-Arg (MMP-1), Pro-Leu-Ala-Tyr-Trp-Ala-Arg (MMP-1), Pro-Leu-Gly-Leu-Trp-Ala-D-Arg-NH2 (MMP-1), Pro-Leu-Gly-Cys(Me)-His-Ala-D-Arg-NH2 (MMP-1) , Pro-Tyr-Ala-Tyr-Trp-Met-Arg (MMP-3), Pro-Leu-Gly-Met-Trp-Ser-Arg (MMP-2 und MMP-9), Pro-Leu-Gly-Leu-Trp-Ala-D-Arg-NH2 (MMP-1) (cleavage at the Gly-Leu site), Pro-β-cyclohexyl-Ala-Gly-Cys (Me)-His-Ala-Lys (N-Methyl-Abz)-NH2 (MMP-1), MeOSuc-Ala-Ala-Pro-Val (Elastase), (N-Acetylglucosamin-Glucuronsäure)ₙ (Hyaluronidase) gewählt wird oder als Substratfragment A ein Oligosachcharid mit mindestens 2 und bis zu 20 Disaccharideinheiten gewählt wird.

Weiterhin ist es von Vorteil, wenn ein Substratfragment mit zwei, drei, vier oder mehr verschiedenen Wirkstoffen verknüpft ist.

Bevorzugt ist auch, wenn solche Wirkkomplexe zusätzlich mit Alkylgruppen, besonders bevorzugt mit C12 bist C18-Alkylgruppen derivatisiert sind und insbesondere Fettsäurereste wie Palmitoyl (C16), Myristyl (C14), Lauryl (C12) oder Stearyl (C18)-Reste enthalten.

Die Erfindung umfasst auch die Verwendung von derartigen Wirkkomplexen zur nicht therapeutischen Behandlung des Menschen sowie topische kosmetische Zubereitungen enthaltend eine oder mehrere der beschriebenen Verbindungen.

Besonders bevorzugt ist es, wenn in topischen kosmetischen Zubereitungen enthaltend eine oder mehrere Verbindungen der Wirkstoffe B zugleich in substratgebundener und nicht substratgebundener Form enthalten ist. Diese Zubereitungen werden bevorzugt zur Behandlung von und zum Schutz vor altersbedingten und/oder UV-induzierten Hautveränderungen (z.B. Sonnenbrand, Altersflecken, photoaging, uneven skin tone, Pigmentierungsstörungen, Schwächung der dermal-epidermalen Junktionszohne, Reduktion der Papillen, Verminderung der mechanischen Belastbarkeit), Hautschäden oder entzündlichen Prozessen (z.B. Heliodermatits, Cuperose), Trockenheit, Rauhigkeit und Schlaffheit der Haut, Faltenbildung, der verminderten Rückfettung durch Talgdrüsen, und einer vergrößerten Anfälligkeit gegenüber mechanischem Streß (Rissigkeit), zur Behandlung von Photodermatosen, den Symptomen der senilen Xerosis und der Lichtalterung (Photoaging) oder aber zum Schutz vor altersbedingten und/oder UV-abhängigen degenerativen Erscheinungen, die mit einem Abbau des Bindegewebes der Haut verbunden sind; besonders bevorzugt dem Schutz vor Abbau von Kollagen, insbesondere von Kollagen 1, 3, 4, 5 und/oder 7; dem Schutz vor Verlust und Erhalt der Hautelastizität/elastischen Fasern; dem Erhalt bzw. die Steigerung der Hautfestigkeit (firming, slimming, contouring, cellular firming), dem Erhalt der Geschmeidigkeit und Feuchtigkeit der Haut verwendet. Dabei ist es erfindungsgemäß, wenn die Zubereitungen in einem beschrifteten Packmittel enthalten sind, dessen Beschriftung auf eine beschriebene Verwendung hinweist.

Die erfindungsgemäße Verwendung von Piqmentierunqsmodulätoren kann einen gleichmäßigen Teint, eine gleichmäßige Hautbräunung (even skin tone), besonders für die Anwendung im Bereich Tanning, Whitening und gegen Altersflecken erzielen.

Bevorzugte Endopeptidasen umfassen vor allem kollagenabbauende-und elastinabbauende Endopeptidasen, insbesondere Matrixmetalloproteinasen (MMPs) und Elastasen sowie hyaluronanabbauende Endo-beta-N-acetylglykosaminidase, besonders bevorzugt Hyaluronidase 2 (HYAL2; AK016575), SPAM1 (s67798), HYAL3 (AF036035), HYAL4 (AF009010) und/oder HYAL5(AF036144).

Zu den bevorzugten MMPs gehören folgende Enzyme, die sich in Kollagenasen und Nicht-Kollagenasen unterteilen:

| | |
|---|---|
| MMP-1 | P03956 (EC 3.4.24.7) |
| MMP-2 | P08253 (EC 3.4.24.24) |
| MMP-3 | P08254 (EC 3.4.24. 17) |
| MMP-7 | P09237 (EC 3.4.24.23) |
| MMP-8 | P22894 (EC 3.4.24.34) |
| MMP-9 | P14780 (EC 3.4.24.35) |
| MMP-10 | P09238 (EC 3.4.24.22) |
| MMP-11 | P24347 (EC 3.4.24) |
| MMP-12 | P39900 (EC 3.4.24.65) |
| MMP-13 | P45452 (EC 3.4.24) |
| MMP-14 | P50281 (EC 3.4.24) |
| MMP-15 | P51511 (EC 3.4.24) |
| MMP-16 | P51512 (EC 3.4.24) |
| MMP-17 | Q9ULZ9 (EC 3.4.24) |
| MMP-19 | Q99542 (EC 3.4.24) |
| MMP-20 | 060882 (EC 3.4.24) |
| MMP-24 | Q9Y5R2 (EC 3.4.24) |
| MMP-25 | Q9NPA2 (EC 3.4.24) |
| MMP-26 | Q9NRE1 (EC 3.4.24) |
| MMP-28 | Q9H239 (EC 3.4.24) |

Die Enzyme MMP 1, 8 und 13 sind Kollagenasen, die übrigen genannten Enzyme Nicht-Kollagenasen. Bei den angegebenen Zahlen handelt es sich um die Zugangsnummern (Accession Numbers) der Swiss-PROT Datenbank des EMBL-EBI (European Bioinforma-tics Institute Heidelberg).

Zu den bevorzugten Elastasen, Elastinabbauenden Endopeptidasen, gehören die Enzyme, die aus dem Pankreas, aus Makrophagen und aus Leukozyten isoliert werden, insbesondere das Enzym Elastase 2, ELA2 (P08246 EC 3.4.21.37).

Zu den bevorzugten Endo-N-acetylglucosaminidasen (das sind Hyaluronidasen) zählen:
SPAM1 (s67798)
HYAL3 (AF036035)
HYAL4 (AF009010)
HYAL5 (AF036144)
und dabei insbesondere HYAL2 (AK016575). Angegeben sind hier die Zugangsnummern der NCBI-Datenbank (National Center for Biotechnology Information) des National Institutes of Health.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

### Vorteilhafte Ausführungsformen

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner, kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine-UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegen-der Erfindung auch in Form commerziell erhältlicher öliger oder wässriger Vordispersionen zur Anwendung kommen. Diesen Vordispersionen können vorteilhaft Dispergierhilfmittel und/oder Solubilisationsvermittler zugesetzt sein.
Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure - | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren-anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCl: Bisoctyltriazol], welches durch die chemische Strukturformel 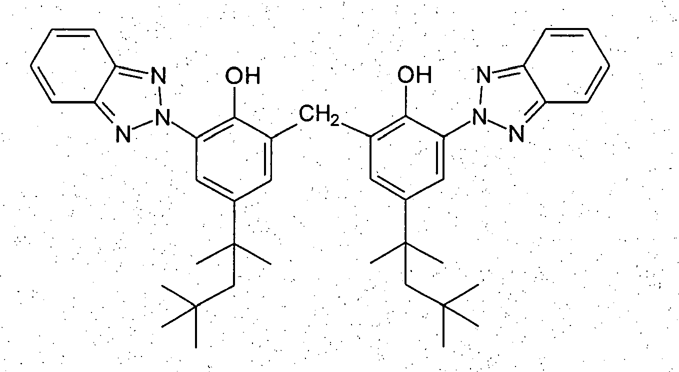 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 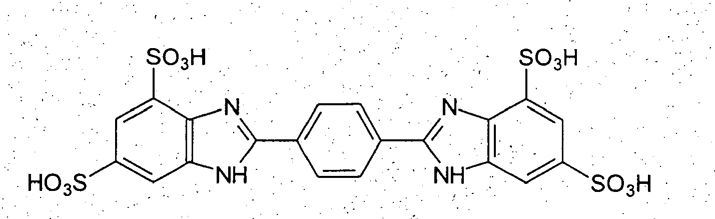 und ihre Salze, besonders die entsprechenden Natrium-, kalium- oder Triethanolammonium-Salze, insbesondere das Phenylen-1,4-bis-(2-benzimidazyl)-3,3'-5,5'-tetrasulfonsäure-bis-natriumsalz 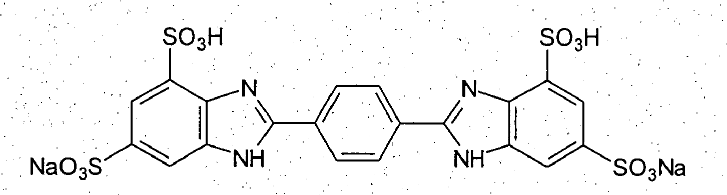 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

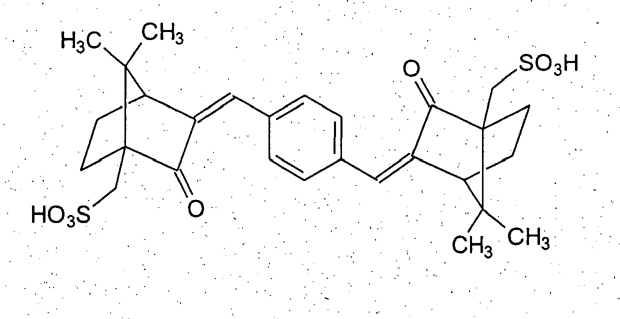

Weitere vorteilhafte UV-A-Filtersübstanzen sind Hydroxybenzophenone, die sich durch die folgende Strukturformel auszeichnen: 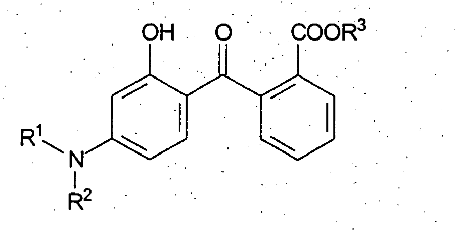 worin
- R¹ und R² unabhängig voneinander Wasserstoff, C₁-C₂₀-Alkyl, C₃-C₁₀-Cydoalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4-Diethylamino-2-hydoxybenzoyl)-benzoesäurehexylester (auch: Aminobenzophenon), welcher sich durch folgende Struktur auszeichnet: 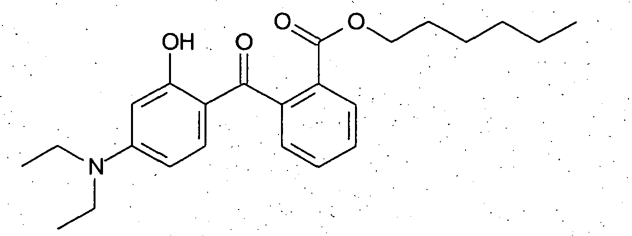 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 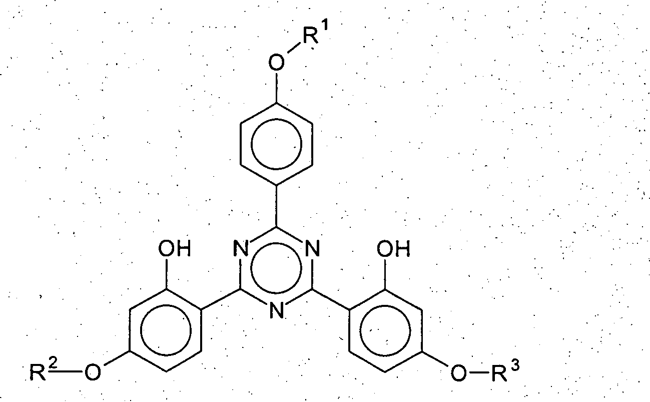 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydihenzoylmethan],Benzotriazolderivate [beispielsweise das 2,2'-Methylen-bis-(6-(2H-benzotriazol-2-yl)-4-(1,1,3,3-tetramethylbutyl)-phenol)],Phenylen-1,4-bis-(2-benzimidazyl)-3,3'-5,5'-tetrasulfonsäureund/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 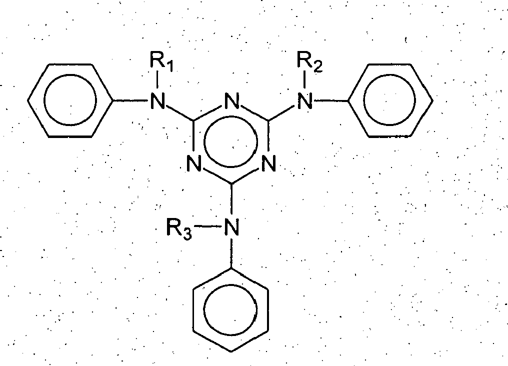 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 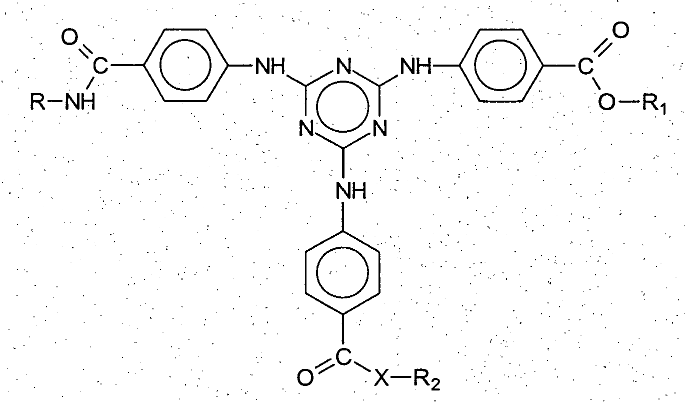 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 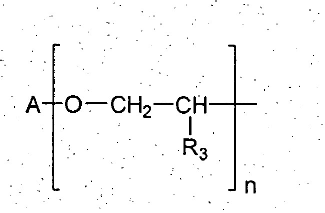 bedeutet, in welcher
A einen verzweigten oder unverzweigten C₁-C₁₈-Alkylrest, einen C₅-C₁₂-Cycloalkyl-oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₈-Alkylrest, einen C₅-C₁₂-Cydoalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₈-Alkylrest, einen C₅-C₁₂-Cycloaikylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 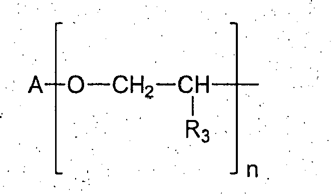 bedeutet, in welcher
A einen verzweigten oder unverzweigten, C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darsfellt.

Besonders bevorzugte UV-Filtersubstanz im Sinne der vorliegenden Erfindung ist ferner ein unsymmetrisch substituiertes s-Triazin, dessen chemische Struktur durch die Formel 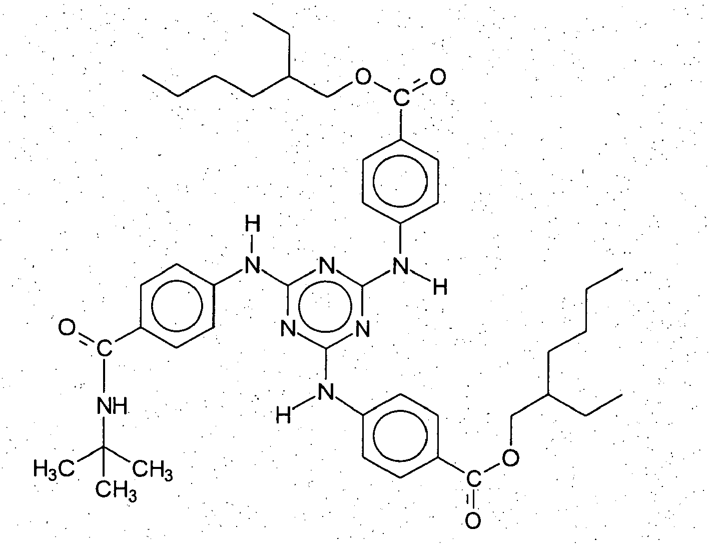 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4'-(1,3,5-Triazin-2,4,6-triyltriimino)-tris-benzoesäure-tris(2-ethylhexylester), synonym: 2,4,6-Tris-[anilino-(p-carbo-2-ethyl-1-hexyloxy)]-1,3,5-triazin(INCl: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 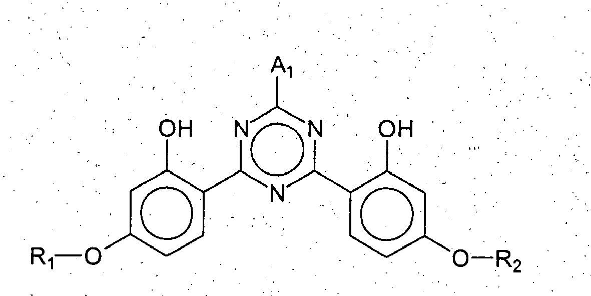 wiedergegeben wird, wobei R₁, R₂ und A₁, verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazinNatriumsalz, das 2,4-Bis-{[4-(3-(2-Propyloxy)-2-hydroxy-propyloxy)-2-hydroxy]-phenyl}-6-(4-methoxy-, phenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1;3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-([4-(2"-methylpropenyloxy)-2-hydroxy]-phenyl)-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 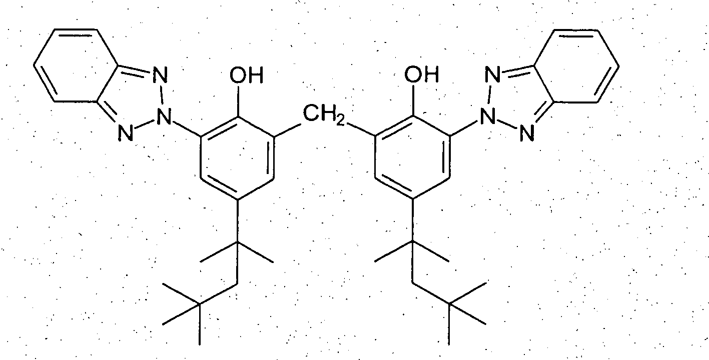 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 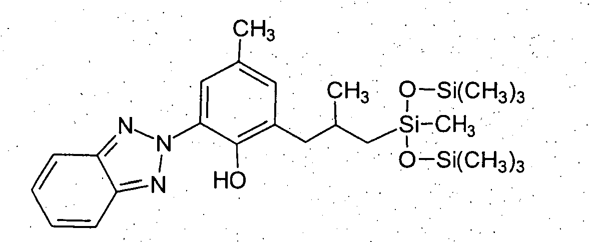 gekennzeichnet ist.

Die UV-B- und/oder Breitband-Filter können öllöslich oder wasserlöslich sein.Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylämino)-benzoesäure(2-ethylhexyl)ester, 4-(Dimethylamino)benzoesäureamylester;
■ 2,4,6-Trianilino-(p-carbo-2-ethyl-1 -hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4 -methylbenzophenon, 2,2 -Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden, Erfindung sind Homomenthylsalicylat (INCl: Homosalate), 2-Ethylhexyl-2-hydroxybenzoat (2-Ethylhexylsalicylat; Octylsalicylat, . INCl: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat,INCl: Octyl Methoxycinnamate) und 4-Methoxyzimtsäureisopentylester (Isopentyl-4-methoxydinnamat, INCl: Isoamyl p-Methoxycinnamate), 3-(4-(2,2-bis Ethoxycarbonylvinyl)-phenoxy)propenyl)-methoxysiloxan/ Dimethylsiloxan - Copolymer (INCl: Dimethicodiethylbenzalmalonat) welches beispielsweise unter der Handelsbezeichnung Parsol® SLX bei Hoffmann La Roche erhältlich ist.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

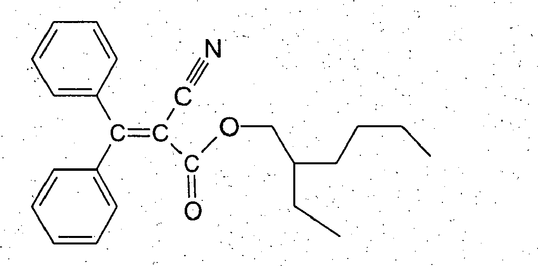

Es kann auch von erheblichem Vorteil sein, polymergebundene oder polymere UV-Filtersubstanzen in Zubereitungen gemäß der vorliegenden Erfindung zu verwenden; insbesondere solche, wie sie in der WO-A-92/20690 beschrieben werden.

Die Liste der genannten ÜV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen,) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-. Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleötid-, Nukleosid-, Peptid- und/oder Lipid-Verbidung) sowie Sulfoximin-verbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid-und/oder Lipid-Verbidung) dieser genannten Wirkstoffe.

Die erfindungsgemäßen Formulierungen können vorteilhaft zusätzlich Wirkstoffe enthalten, die den Zustand der Haut positiv beeinflussen. So zeigte sich, dass Wirkstoffe zur positiven Beeinflussung der Altershaut, die die Entstehung von Falten oder auch bestehenden Falten vermindern (Biochinone, insbesondere Ubichinon Q10,) oder die Restrukturierung des Bindegewebes fördern (Isoflavonoide), in den erfindungsgemäßen Formulierungen sehr gut verwendet werden können. Auch zeigte sich, dass sich die Formulierungen in besonderer Weise eignen, Wirkstoffe zur Unterstützung der Hautfunktionen bei (alters-) trockener Haut (Serinol, Osmolyte wie Taurin etc.) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Dexpanthenol, Inhibitoren des Prostaglandinstoffwechsels (insbesondere der Cyclooxygenase) und des Leukotrienstoffwechsels (insbesondere der 5-Lipoxygenase). Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen (Tyrosinsulfat, Dioic acid (8-Hexadecen-1,16-dicarbonsäure), Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte). In gleicher Weise erwiesen sich die erfindungsgemäßen Formulierungen als hervorragende Grundlage für Wirkstoffe, die eine verstärkte/ schnellere Bräunung der Haut herbeiführen (Advanced Glycation Endproducts (AGE), Lipofuscine, Nukleinsäure-Oligonukleotide, Purine und Pyrimidine, NO-freisetzende Substanzen), sei es mit oder ohne Einfluss von UV-Licht.

Erfindungsgemäße Zubereitungen können auch vorteilhaft Verdicker enthalten. Geeignete Verdicker sind:

Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX.

Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum.

Außerdem geeignet sind Copolymere der Acrylsäure oder der Methacrylsäure wie z.B. Carbopol 1342 oder Permulen TRI.

Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

Erfindungsgemäße Zubereitungen können auch vorteilhaft Cyclodextrine (Cycloamylosen, Cycloglucane) enthalten.

Die Verbesserung der Löslichkeit schwerlöslicher Substanzen in Gegenwart von Cyclodextrinen in wässrigem Milieu ist für einzelne Substanzen bekannt. Vorteilhaft können sowohl die Einschlußverbindungen einer Substanz, auch Gast genannt, mit einer Cyclodextrinspezies, wobei sowohl 1:1 oder 1:2 Komplexe, wie auch Komplexe mit weiteren molaren Verhältnissen (Gast: Cyclodextrin) möglich sind, sowie auch deren physikalische Mischung sein.

Es handelt sich bei den Cyclodextrinen um zyklische Oligosaccharide bestehend aus α-1,4 verknüpften Glucosebausteinen. In der Regel sind sechs bis acht Glucosebausteine (α-, β-, bzw. γ-Cyclodextrin) miteinander verbunden.

Cyclodextrine werden bei Einwirkung von *Bacillus macerans* auf Stärke erhalten. Sie besitzen einen hydrophoben Innenraum und eine hydrophile Außenseite. Cyclodextrine und ihre Derivate können aufgrund Ihrer Struktur Inklusionskomplexe bilden. Sie sind zur "molekularen Verkapselung" von Wirkstoffen geeignet (z.B. als schützende Umhüllung empfindlicher Moleküle in kosmetischen und pharmazeutischen Formulierungen).

Diese Anwendungen sind auch in einer Reihe von Patenten beschrieben. (z.B.:.WO 98/55148, EP 0 579 435, EP 0 392 608). In diesen Schriften wird jedoch meist nur ein Wirkstoff vom Cyclodextrin (-derivat) komplexiert. Mehrfachkomponenten-Inklusionskomplexe werden zwar in EP 0756 493 beschrieben, doch handelt es sich hier bei näherer Betrachtung um ein Salz und nicht um eine Zweikomponentenmischung von Säure und Base.

Mit "Cyclodextrin und/oder ein Derivat davon" sind im folgenden sowohl Cyclodextrine mit unterschiedlicher Anzahl von Glucosebausteinen im Ringmolekül als auch Derivate dieser Verbindungen gemeint.

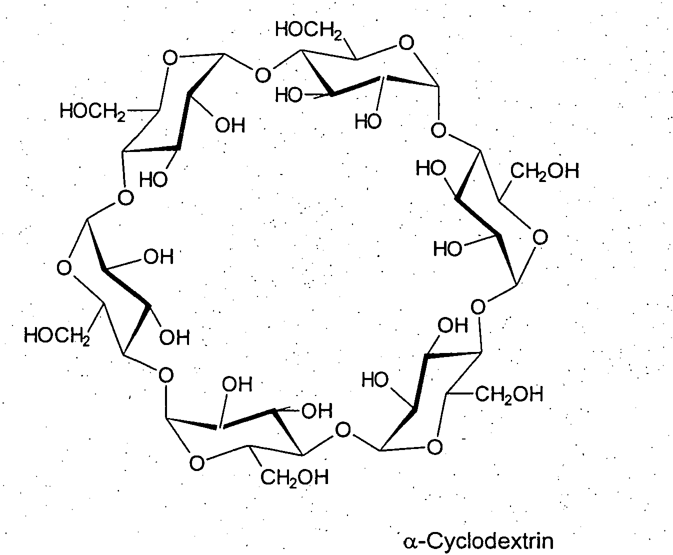

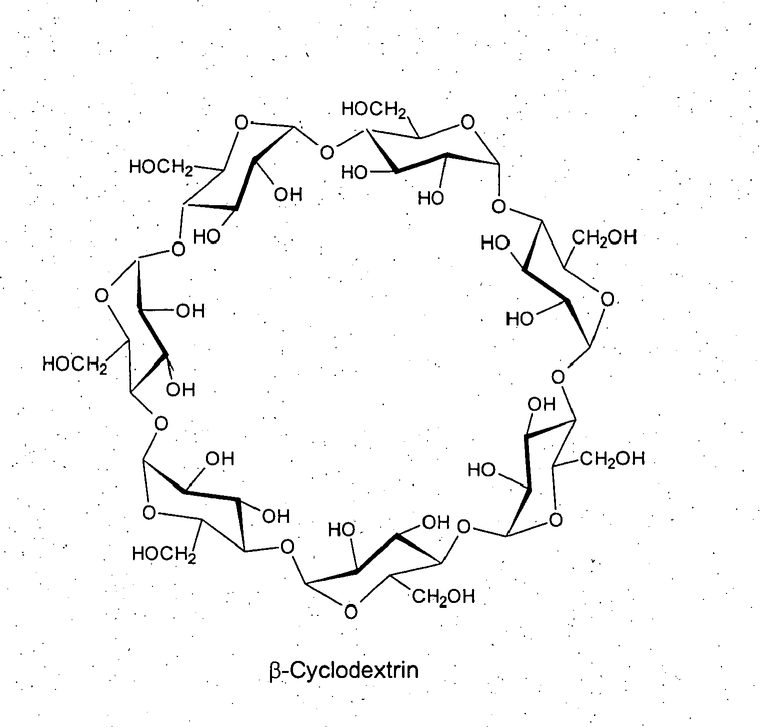

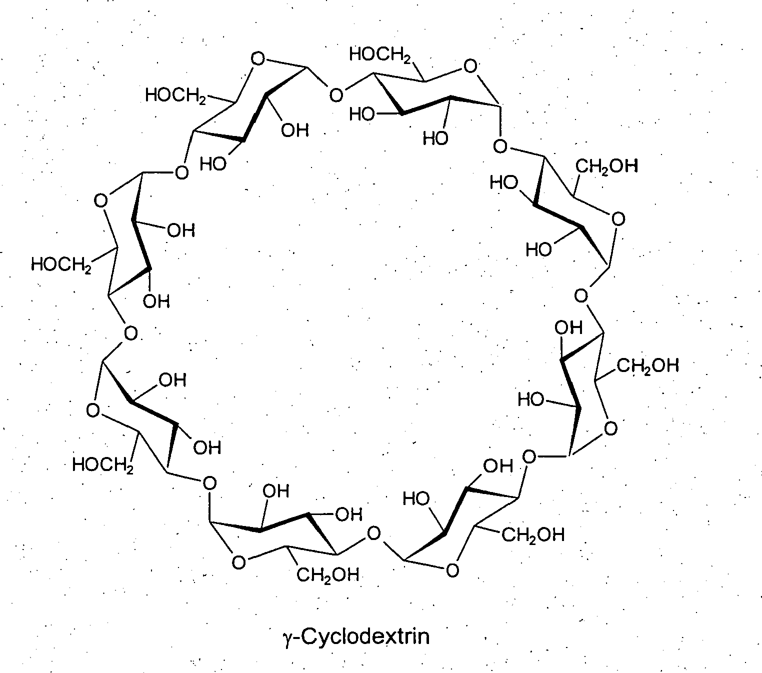

Erfindungsgemäß werden das oder die Cyclodextrine bevorzugt in kosmetischen oder dermatologischen Zusammensetzungen eingesetzt in einer Konzentration von 0.0005 bis 20.0 Gewichts-%, insbesondere 0,01 bis 10 Gew.- % und besonders bevorzugt in einer Konzentration von 0.1 bis 5.0 Gew.-%.

Es ist erfindungsgemäß vorteilhaft native, polar- und/oder unpolar- substituierte Cyclodextrine einzusetzen. Hierzu gehören vorzugsweise aber nicht ausschließlich Methyl-, insbesondere random-Methyl-β-Cyclodextrin, Ethyl- sowie Hydroxypropyl-Cyclodextrine, beispielsweise HP-β-Cyclodextrin oder HP-γ-Cyclodextrin.

Die erfindungsgemäß besonders bevorzugten Cyclodextrinspezies sind γ-Cyclodextrin sowie Hydroxypropyl-β-Cylcodextrin.

Ein weiterer Stand der Technik ist in folgenden Schriften enthalten:
K. Uekama et al., Chemical Reviews, 1998, 98, 2045-2076, "Cyclodextrin drug carrier systems"
T. Loftsson, Int. J. Dermatology, 1998, 37, 241-246, "Cyclodextrins: new drugdelivery systems in dermatology".
J. Zatz et al. Cosmetics & Toiletries, 1997, 112, Juli, S. 39ff, "Applications of cyclodextrins in skin products.
U. Citernesi, Cosmetics & Toiletries, 1995, 110, März, S. 53 ff, Cyclodextrins in functional dermocosmetics.

### Bücher:

### J. Szejtli, "Cyclodextrin Technology", Kluwer, Dordrecht, 1988

Comprehensive Supramolecular Chemistry, Vol 3, "Cyclodextrins, Pergamon, 1996.
Die erfindungsgemäß verwendeten Cyclodextrine bzw. Cyclodextrin-Gast-Inklusionskomplexe bzw. die Cyclodextrin-Substanz Mischungen lassen sich ohne Schwierigkeiten in gängige kosmetische oder dermatologische Formulierungen einarbeiten, vorteilhaft in Cremes (z.B. O/W, W/O/W, W/O), Salben, Hydrödispersionen, Lotionen, Tinkturen, Pumpspräys, Aerosolsprays, wässrige Lösungen und dergleichen. Eine Anwendung kann auf der Haut, der Kopfhaut, unter der Achsel oder am Haar erfolgen. In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat. | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konervierungsmittel oder Konservierungshilfsstoffe Dibromdicyanobutan (2-Brom-2-brommethylglutarodinitril), 3-lod-2-propinylbutylcarbamat, 2-Brom-2-nitro-propan-1;3-diol, Imidazolidinylharnstoff, 5-Chlor-2-methyl-4-isothiazolin-3-on, 2-Chloracetamid, Benzalkoniumchlorid, Benzylalkohol, DMDM Hydantoin , IPBC (Formaldehydabspalter) geeignet.

Ferner sind Phehylhydroxyalkylther, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186; DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-4324 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Vorteilhafte Emulgatoren sind beispielsweise Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylen Glycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; lsostearylglycerylether; Cetylstearylalkohol,im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/C₁₀₋₃₀-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Die in den Beispielen angeführten Wirkkomplexe sind wie folgt definiert:

Der Wirkkomplex enthält die Substrukturen A und B, wobei jeweils mindestens ein A und B durch mindestens eine kovalente Bindung verbunden sind und A ein durch Stress aktivierbares hautenzymabspaltbares Substratfragment ist und B einen kosmetischen und/oder dermatologischen Wirkstoff darstellt. Dabei kann die kovalente Bindung des Wirkkomplexes beispielsweise eine Ester-, Acetal-, Peptid- oder Amidbindung sein. Die Wirkkomplexe können durch stress aktivierbare Hautenzyme wie z.B. Elastasen, Hyaluronidasen oder Metalloproteinasen aktiviert werden. Als Wirkstoff B des Wirkkomplexes kommen Hydroxyl-, Carboxyl-, Keto- oder Aminogruppen tragende Antifalten- oder Lichtschutzmittel, Antioxidantien, Pigmentierungsmodulatoren (Tanning, whitening, Altersflecken), anti-entzündliche Wirkstoffe, Hautbefeuchter oder Energisatoren in Frage. Ein Hydroxylgruppen tragender Wirkstoff B ist z.B. ein Isoflavonoid, bevorzugt Genistein, Sylimarin, Daidzein, Arctiin, Arctigenin, Ubiquinol (reduziertes Q10), Mevalonsäure, ein Vitamin, besonders bevorzugt Vitamin A, C oder E, Cholesterin und/oder Licochalcon A. Ein Carboxylgruppen tragender Wirkstoff B kann aus der Gruppe um Kreatin, Mevalonsäure und Vitamin C gewählt werden. Als ein Ketogruppen tragender Wirkstoff B können Isoflavonoide, insbesondere Genistein, Arctiin, Arctigenin, Q10, Peptidische Wirkstoffe (insbesondere Tri- bis Decapeptide), Licochalcone A, Liponamid und/oder Taurin gewählt werden. Als Aminogruppen tragender Wirkstoff B kommen zudem Kreatin, ein peptidischer Wirkstoff, besonders bevorzugt Tri-bis Decapeptide, Liponamid und/oder Taurin in Frage.

Als Substratfragnient A kann ein Hydroxyl-, Carboxyl-, Keto- oder Aminogruppen tragendes Substratfragment verwendet werden.

### Beispiel A

### Herstellung von Kreatin-Pro-Leu-Ala-Tyr-Trp-Ala-Arg-Lys-Thr-Thr-Lys-Ser

Kreatin-Pro-Leu-Ala-Tyr-Trp-Ala-Arg-Lys-Thr-Thr-Lys-Ser stellt einen durch MMP-1 aktivierbaren erfindungsgemäßen Energisator dar.

### Das 12mer wird durch eine Peptidsynthese an fester Phase dargestellt:

Die Synthese der linearen Peptidbausteine wird nach der von Merrifield 1963 entwickelten Festphasensynthese an einem unlöslichen, polymeren Träger (mit 1% m-Divinylbenzol quervernetztes Polystyrol-Harz) durchgeführt. Als Linker dient Tritylchlorid.

Die Peptid-Synthese erfolgt nach der Fmoc-Strategie (Fmoc = 9-Fluorenylmethoxycarbonyl).

Zur Synthese werden basenlabile, N-terminal Fmoc geschützte Aminosäuren verwendet. Als permanente Schutzgruppen werden Aminosäuren mit säuresensitiven tert-Butyl bzw. Boc-Schutzgruppen eingesetzt (Boc- = tert-Butyloxycarboyl-).

Als erste Aminosäure wird Serin in wasserfreiem Dichlormethan unter Zugabe von DIPEA an das Harz gekuppelt (DIPEA = Diisopropylethylamin).

Nachfolgend wird jeweils erst die N-terminale Fmoc-Schutzgruppe abgespalten. Der nächste Schritt beinhaltet die Kupplung der nächsten Aminosäure der oben genannten Sequenz. Dieser Abspaltung-/Kupplungsprozeß wird den 11 Aminosäuren wiederholt.
Die Fmoc-Entschützungen erfolgen durch Zugabe von 20% Piperidin in NMP (NMP = N-Methylpyrrolidon).

Die Kupplungsreaktionen erfolgen nach der TBTU/HOBt-Methode mit der Base DIPEA in NMP (TBTU=2-(1H-Benzotrizol-1-yl)-1,1,3,3-tetramethyluronoim tetrafluoroborat,HOBt = Hydroxybenzotriazol).

An das N-terminale Ende des 12-mers wird durch eine weitere Kupplungsreaktion mit der Carboxyfunktionalität Kreatin (ungeschützt) geknüpft.

Die Abspaltung des "Kreatin-12-mers" von der festen Phase sowie die Abspaltung aller permanenten Schutzgruppen erfolgt durch Reaktion mit TFA unter Verwendung von Scavenger Kationenfängern (Thiomethanol, Thioanisol, Ethandithiol).

Zu Modulation des logP-Wertes können im Anschluß an die Abspaltung Palmitoyl-Reste-durch Reaktion mit Palmitoylchorid eingefügt werden (Reaktion der primären Aminofunktionen der Seitenketten der Lysin-Bausteine).

Analoge aktivierbare Wirkstoffe werden hergestellt, indem man Kreatin durch tsoftavonoide, Sylimarin, Arctiin, Arctigenin, Ubiquinol, Mevälonsäure, Vitamine, Licochalcon A, Peptide, Liponamid oder Taurin ersetzt.

### 1. Beispiele O/W-Creme

| **Beispiele** | 1 | 2 | 3 | 4 | 5 |
|---|---|---|---|---|---|
| Glycerylstearatcitrat | | | 2,00 | | 2,00 |
| Glycerylsterat selbstemulgierend | 4,00 | 3,00 | | | |
| PEG-40-Stearat | 1,00 | | | | |
| Polyglyceryl-3-Methylglucose-Distearat | | | | 3,00 | |
| Sorbitanstearat | | | | | 2,00 |
| Stearinsäure | | 1,00 | | | |
| Stearylalkohol | | | 5,00 | | |
| Cetylalkohol | 3,00 | 2,00 | | 3,00 | |
| Cetylstearylalkohol | | | | | 2,00 |
| Caprylic-/Capric-Triglycerid | 5,00 | 3,00 | 4,00 | 3,00 | 3,00 |
| Octyldodecanol | | | 2,00 | | 2,00 |
| Dicaprylylether | | 4,00 | | 2,00 | 1,00 |
| Paraffinum liquidum | 5,00 | 2,00 | | 3,00 | |
| Titandioxid | | | 1,00 | | |
| 4-Methylbenzyliden Campher | | | 1,00 | | |
| 1-(4-tert-Butylphenyl)-3-(4-methoxyphenyl)-1,3-propandion | | | 0,50 | | |
| Wirkkomplex aus Beispiel A | 0,20 | 0,50 | 0,10 | 1,00 | 0,30 |
| Wirkstoff | | 0,50 | | | 1,00 |
| Tocopherol | 0,1 | | | | 0,20 |
| Biotin | | | 0,05 | | |
| Ethylendiamintetraessigsaeure Trinatrium-Salz | 0,1 | | 0,10 | 0,1 | |
| Konservierungsmittel | q.s. | q.s. | q.s. | q.s. | q.s. |
| Xanthan Gummi | | | | | |
| Polyacrylsaeure | 3,00 | 0,1 | | 0,1 | 0,1 |
| Natronlauge 45% | q.s | q.s. | q.s. | q.s. | q.s. |
| Glycerin | 5,00 | 3,00 | 4,00 | 3,00 | 3,00 |
| Butylenglycol | | 3,00 | | | |
| Parfum | q.s. | q.s. | q.s. | q.s. | q.s. |
| Wasser | ad 100 | Ad 100 | Ad 100 | Ad 100 | Ad 100 |

### 2. Beispiele O/W-Creme

| **Beispiele** | **1** | **2** | **3** | **4** | **5** |
|---|---|---|---|---|---|
| Glycerylstearatcitrat | | 2,00 | 2,00 | | |
| Glycerylsterat selbstemulgierend | 5,00 | | | | |
| Stearinsäure | | | | 2,50 | 3,50 |
| Stearylalkohol | 2,00 | | | | |
| Cetylalkohol | | | | 3,00 | 4,50 |
| Cetylstearylalkohol | | 3,00 | 1,00 | | 0,50 |
| C12-15 Alkylbenzoat | | 2,00 | 3,00 | | |
| Caprylic-/Capric-Triglycerid | 2,00 | | | | |
| Octyldodecanol | 2,00 | 2,00 | | 4,00 | 6,00 |
| Dicaprylylether | | | | | |
| Paraffinum liquidum | | 4,00 | 2,00 | | |
| Cyclisches Dimethylpolysiloxan | | | | 0,50 | 2,00 |
| Dimethicon Polydimethylsiloxan | 2,00 | | | | |
| Titandioxid | 2,00 | | | | |
| 4-Methylbenzyliden Campher | 1,00 | | | | 1,00 |
| 1-(4-tert-Butylphenyl)-3-(4-methoxyphenyl)-1,3-propandion | 0,50 | | | | 0,50 |
| Wirkkomplex aus Beispiel A | 0,20 | 0,70 | 0,25 | 1,00 | 0,40 |
| Wirkstoff | 0,30 | 1,50 | | | 0,75 |
| Tocopherol | | | | | 0,05 |
| Ethylendiamintetraessigsaeure Trinatriumsalz | | | 0,20 | | 0,20 |
| Konservierungsmittel | q.s. | q.s. | q.s. | q.s. | q.s. |
| Xanthan Gummi | | | 0,20 | | |
| Polyacrylsaeure | 0,15 | 0,1 | | 0,05 | 0,05 |
| Natronlauge 45% | q.s. | q.s. | q.s. | q.s. | q.s. |
| Glycerin | 3,00 | | 3,00 | 5,00 | 3,00. |
| Butylenglycol | | 3,00 | | | |
| Ethanol | | 3,00 | | 3,00 | |
| Parfum | q.s. | q.s. | q.s. | q.s. | q.s. |
| Wasser | Ad 100 | Ad 100 | Ad 100 | Ad 100 | Ad 100 |

### 3. Beispiele W/O-Emulsionen

| **Beispiele** | **1** | **2** | **3** | **4** | **5** |
|---|---|---|---|---|---|
| Cetyldimethiconcopolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| 2-Ethylhexyl Methoxyzinnamat | | 8,00 | | 5,00 | 4,00 |
| 2,4-Bis-(4-(2-ethyl-hexyloxy-)2-hydroxyl)-phenyl)-6-(4-methoxyphenyl)-(1,3,5)-triazin | 2,00 | 2,50 | | 2,00 | 2,50 |
| 1-(4-tert-Butylphenyl)-3-(4-methoxyphenyl)-1,3-propandion | | | 2,00 | 1,00 | |
| Diethylhexyl Butamidotriazon | 3,00 | 1,00 | | | 3,00 |
| Ethylhexyl Triazon | | | 3,00 | 4,00 | |
| 4-Methylbenzyliden Campher | | 2,00 | | 4,00 | 2,00 |
| Octocrylen | 7,00 | 2,50 | 4,00 | | 2,50 |
| Diethylhexyl Butamidotriazon | 1,00 | | | 2,00 | |
| Phenylen-1,4-bis-(mononatrium, 2-benzimidazyl-5,7-disulfonsaeure) | 1,00 | 2,00 | 0,50 | | |
| Phenylbenzimidazol Sulfonsäure | 0,50 | | | 3,00 | 2,00 |
| Titandioxid | | 2,00 | 1,50 | | 3,00 |
| Zinkoxid | 3,00 | 1,00 | 2,00 | 0,50 | |
| Paraffinum liquidum | | | 10,0 | | 8,00 |
| C12-15 Alkyl-Benzoat | | | | 9,00 | |
| Dicaprylylether | 10,00 | | | | 7,00 |
| Butylen-Glycol-Dicaprylat/-Dicaprat | | | 2,00 | 8,00 | 4,00 |
| Dicaprylylcarbonat | 5,00 | | 6,00 | | |
| Dimethicon Polydimethylsiloxan | | 4,00 | 1,00 | 5,00 | |
| Phenylmethylpolysiloxan | 2,00 | 25,00 | | | 2,00 |
| Shea Butter | | | 3,00 | | |
| PVP Hexadecencopolymer | 0,50 | | | 0,50 | 1,00 |
| Octoxyglycerin | | 0,30 | 1,00 | | 0,50 |
| Glycerin | 3,00 | 7,50 | | 7,50 | 2,50 |
| Glycinsoja | | 1,00 | 1,50 | | |
| Magnesiumsulfat | 1,00 | 0,50 | | 0,50 | |
| Magnesiumchlorid | | | 1,00 | | 0,70 |
| Tocopherolacetat | 0,50 | | 0,25 | | 1,00 |
| Wirkkomplex aus Beispiel A | 0,10 | 0,60 | 1,00 | 1,00 | 0,80 |
| Wirkstoff | 0,90 | 0,10 | | | 1,00 |
| Konservierungsmittel | q.s. | q.s. | q.s. | q.s. | q.s. |
| Ethanol | 3,00 | | 1,50 | | 1,00 |
| Parfum | q.s. | q.s. | q.s. | q.s. | q.s. |
| Wasser | ad. 100 | ad. 100 | ad. 100 | ad. 100 | ad. 100 |

### 4.Beispiele W/O Emulsionen

| **Beispiele** | **1** | **2** |
|---|---|---|
| Polyglyceryl-2-dipolyhydroxystearat | 4,00 | 5,00 |
| PEG-30-dipolyhydroxystearat | | |
| Lanolinalkohol | 0,50 | 1,50 |
| Isohexadecan | 1,00 | 2,00 |
| Myristyl-Myristat | 0,50 | 1,50 |
| Vaseline | 1,00 | 2,00 |
| 1-(4-tert-Butylphenyl)-3-(4-methoxyphenyl)-1,3-propandion | 0,50 | 1,50 |
| 4-Methylbenzyliden Campher | | 3,00 |
| Butylen-Glycol-Dicaprylat/-Dicaprat | 4,00 | 5,00 |
| Shea Butter | | 0,50 |
| Butylenglycol | | 6,00 |
| Octoxyglycerin | | 3,00 |
| Glycerin | 5,00 | |
| Tocopherolacetat | 0,50 | 1.,00 |
| Wirkkomplex aus Beispiel A | 0,20 | 0,45 |
| Wirkstoff | 0,90 | |
| Ethylendiamintetraessigsaeure | 0,20 | 0,20 |
| Konservierungsmittel | q.s. | q.s. |
| Ethanol | | 3,00 |
| Parfum | q.s. | q.s. |
| Wasser | ad. 100 | ad.100 |

### 5.Beispiele Hydrodispersionen

| **Beispiele** | **1** | **2** | **3** | **4** | **5** |
|---|---|---|---|---|---|
| Polyoxyethylen(20)cetylstearylether | 1,00 | | | 0,5 | |
| Cetylalkohol | | | 1,00 | | |
| Natriumpolyacrylat | | 0,20 | | 0,30 | |
| Acrylate /C10-30-Alkyl-Acrylat Cross-polymer | 0,50 | | 0,40 | 0,10 | 0,10 |
| Xanthan Gummi | | 0,30 | 0,15 | | 0,50 |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | 1,00 | | 2,00 | | |
| Diethylhexyl Butamidotriazon | | 2,00 | | 2,00 | 1,00 |
| Ethylhexyl Triazon | 4,00 | | 3,00 | 4,00 | |
| 4-Methylbenzyliden Campher | | | | | 2,00 |
| Octocrylen | | 4,00 | 4,00 | | 2,50 |
| Phenylen-1,4-bis-(mononatrium, 2-benzimidazyl-5,7-disulfonsaeure | 1,00 | | 0,50 | | 2,00 |
| Phenylbenzimidazol Sulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| C12-15 Alkyl Benzoat | 2,00 | 2,50 | | | |
| Dicaprylylether | | 4,00 | | | |
| Butylenglycol-Dicaprylat/-Dicaprat | 4,00 | | 2,00 | 6,00 | |
| Dicaprylylcarbonat | | 2,00 | 6,00 | | |
| Dimethicon (Polydimethylsiloxan) | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| Wirkkomplex aus Beispiel A | 0,15 | 0,60 | 1,00 | 1,00 | 0,80 |
| Wirkstoff | 1,25 | | 0,10 | | 0,15 |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad. 100 | ad. 100 | ad. 100 | ad. 100 | ad. 100 |

### 6.Beispiel (Gelcreme)

| | |
|---|---|
| Acrylat/C10-30 Alkylacrylat Crosspolymer | 0,40 |
| Polyacrylsaeure | 0,20 |
| Xanthan Gummi | 0,10 |
| Cetearylalkohol | 3,00 |
| C12-15 Alkylbenzoat | 4,00 |
| Caprylic/Capric Triglycerid | 3,00 |
| Cyclisches Dimethylpolysiloxan | 5,00 |
| Dimeticon Polydimethylsiloxan | 1,00 |
| Wirkkomplex.aus Beispiel A | 0,05 |
| Wirkstoff | 1,00 |
| Glycerin | 3,00 |
| Natriumhydroxid | q.s. |
| Konservierungsmittel | q.s. |
| Parfum | q.s. |
| Wasser | ad 100,0 |
| pH-Wert eingestellt auf 6.0 | |

### 7.Beispiel(W/O-Creme)

| | |
|---|---|
| Polyglyceryl-3-Diisostearate | 3,50 |
| Glycerin | 3,00 |
| Polyglyceryl-2-Dipolyhydroxystearate | 3,50 |
| Wirkkomplex aus Beispiel A | 0,60 |
| Wirkstoff | 1,00 |
| Konservierungsmittel | q.s. |
| Parfum | q.s. |
| Wasser | ad 100,0 |
| Magnesiumsulfat | 0,6 |
| Isopropylstearat | 2,0 |
| Caprylylether | 8,0 |
| Cetearylisononanoat | 6,0 |

### 8.Beispiel (W/O/W-Creme):

| | |
|---|---|
| Glycerylstearat | 3,00 |
| PEG-100-Stearat | 0,75 |
| Behenylalkohol | 2,00 |
| Caprylic-/Capric-Triglycerid | 8,0 |
| Octyldodecanol | 5,00 |
| C12-15 Alkylbenzoat | 3,00 |
| Wirkkomplex aus Beispiel A | 0,50 |
| Wirkstoff | 0,65 |
| Magnesiumsulfat (MgSO4) | 0,80 |
| Ethylendiamintetraessigsaeure | 0,10 |
| Konservierungsmittel | q.s. |
| Parfum | q.s. |
| Wasser | ad 100,0 |
| pH-Wert eingestellt auf 6.0 | |

### 9. Beispiel (Duschbad)

| | |
|---|---|
| Natriumlaurethsulfat | 33,00 |
| Kalium-Cocoyl-hydrolysiertes Kollagen (30%) | 11,00 |
| Cocoamphodiacetat (30%) | 5,00 |
| PEG-7-Glyceryl Cocoat | 2,00 |
| Cocamid MEA | 1,00 |
| Natriumchlorid | 0,50 |
| Wirkkomplex aus Beispiel A | 0,05 |
| Wirkstoff | 0,35 |
| Zitronensäure | 0,02 |
| Konservierungsmittel, Farbstoffe, Parfüm | q.s. |
| Wasser | ad 100 |

### 10. Beispiel (Haarkur)

| | |
|---|---|
| Hydroxypropylmethylcellulose | 0,50 |
| Cetrimoniumbromid | 1,00 |
| Glycerin | 3,00 |
| Cetearylalkohol | 2,50 |
| Benzophenon-4 | 0,4 |
| Glycerylstearat | 2,00 |
| Wirkkomplex aus Beispiel A | 0,1 |
| Wirkstoff | 0,20 |
| Konservierungsmittel, Parfüm, pH-Einstellung | q.s. |
| Wasser | ad100 |
| Der pH-Wert wird auf 3,5 eingestellt. | |

### 11. Beispiel (Haarspülung)

| | |
|---|---|
| Behentrimoniumchlorid | 1,00 |
| Glycerin | 3,00 |
| Benzophenon-4 | 0,25 |
| Hydroxyethylcellulose | 0,20 |
| Cetearylalkohol | 3,00 |
| Wirkkomplex aus Beispiel A | 0,2 |
| Wirkstoff | 0,15 |
| Folsäure | 0,80 |
| Konservierungsmittel, Parfüm, pH-Einstellung | q.s. |
| Wasser | ad 100 |
| Der pH-Wert wird auf 3,0 eingestellt. | |

### 12. Beispiel (Conditioner-Shampoo mit Perlglanz)

| | 1 | 2 | 3 |
|---|---|---|---|
| Polyquaternium-10 | 0,5 | 0,5 | 0,5 |
| Natriumlaurethsulfat | 9,0 | 9,0 | 9,0 |
| Benzophenon-3 | | 0,5 | |
| Benzophenon-4 | | | 0,4 |
| Cocoamidopropylbetain | 2,5 | 2,5 | 2,5 |
| Perlglanzmittel | 2,0 | 2,0 | 2,0 |
| Wirkkomplex aus Beispiel A | 0;06 | 0,15 | 0,01 |
| Wirkstoff | 0,15 | | 0.05. |
| Dinatrium EDTA | 0,1 | 0,2 | 0,15 |
| Konvervierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |
| Der pH-Wert wird auf 6 eingestellt. | | | |

### 13. Beispiel (klares Conditioner-Shampoo)

| **Beispiel** | **1** | **2** | **3** |
|---|---|---|---|
| Polyquaternium-10 | 0,5 | 0,5 | 0,5 |
| Benzophenon-4 | | 0,4 | |
| 2-Ethylhexyl Methoxyzinnamat | | | 0,2 |
| Natriumlaurethsulfat | 9,0 | 9,0 | 9,0 |
| Cocoamidopropylbetain | 2,5 | 2,5 | 2,5 |
| Wirkkomplex aus Beispiel A | 0,02 | 0,05 | 0,05 |
| Wirkstoff | 0,05 | | 0,10 |
| Iminodibernsteinsäure, Na-Salz | 0,2 | 0,3 | 0,8 |
| Konvervierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |
| Der pH-Wert wird auf 6 eingestellt. | | | |

### 14. Beispiel (klares Light-Shampoo mit Volumeneffekt)

| **Beispiel** | **1** | **2** | **3** |
|---|---|---|---|
| Natriumlaurethsulfat | 10,0 | 10,0 | 10,0 |
| Cocoamidopropylbetain | 2,5 | 2,5 | 2,5 |
| Wirkkomplex aus Beispiel A | 0,50 | 0,60 | 0,30 |
| Wirkstoff | 0,20 | | 0,10 |
| Dinatrium EDTA | 0,2 | 0,15 | 0,7 |
| Konvervierungsmittel, Parfüm, Verdicker, pH-Einstellung und Lösungsvermittler | q.s | q.s. | q.s |
| Wasser, VES (vollentsalzt) | ad 100,0 | ad 100,0 | ad 100,0 |
| Der pH-Wert wird auf 5,5 eingestellt. | | | |

## Patentansprüche

1. Wirkkomplex enthaltend die Substrukturen A und B, wobei jeweils mindestens ein A und B durch mindestens eine kovalente Bindung verbunden sind und A ein durch Stress aktivierbare Hautenzyme abspaltbares Substratfragment ist und B ein kosmetischer und/oder dermatologischer Wirkstoff.

2. Wirkkomplex nach Patentanspruch 1 **dadurch gekennzeichnet, daß** die kovalente Bindung eine Ester-, Acetal-, Peptid- oder Amidbindung darstellt.

3. Wirkkomplexe nach einem der vorangehenden Ansprüche **dadurch gekennzeichnet, daß** das durch Stress aktivierbare Hautenzym eine Elastase, Hyaluronidase oder Metalloproteinase darstellt.

4. Wirkkomplexe nach einem der vorangehenden Ansprüche **dadurch gekennzeichnet, daß** als Wirkstoff B ein Hydroxyl-, Carboxyl-, Keto- oder Aminogruppen tragendes Antifatten- oder Lichtschutzmittel, Antioxidans, ein Pigmentierungsmodulator, ein anti-entzündlicher Wirkstoff, ein Hautbefeuchter oder ein Energisator gewählt wird.

5. Wirkkomplexe nach einem der vorangehenden Ansprüche **dadurch gekennzeichnet, daß** als Hydroxylgruppen tragender Wirkstoff B ein Isoflavonoid, bevorzugt Genistein, Sylimarin, Daidzein, Arctiin, Arctigenin, Ubiquinol, Mevalonsäure, ein Vitamin, besonders bevorzugt Vitamin A, C oder E, Cholesterin und/oder Licochalcon A gewählt wird.

6. Wirkkomplexe nach einem der vorangehenden Ansprüche **dadurch gekennzeichnet, daß** als Carboxylgruppen tragender Wirkstoff B Kreatin, Mevalonsäure und Vitamin C gewählt wird.

7. Wirkkomplexe nach einem der vorangehenden Ansprüche **dadurch gekennzeichnet, daß** als Ketogruppen tragender Wirkstoff B Isoflavonoidä, insbesondere Genistein, Arctiin, Arctigenin, Q10, Peptidische Wirkstoffe, Licochalcone A, Liponamid und/oder Taurin gewählt wird.

8. Wirkkomplexe nach einem der vorangehenden Ansprüche **dadurch gekennzeichnet, daß** als Aminogruppen tragender Wirkstoff B Kreatin, ein peptidischer Wirk-Prtpl-02163 stoff, besonders bevorzugt Tri- bis Decapeptide, Liponamid und/oder Taurin gewählt wird.

9. Wirkkomplexe nach einem der vorangehenden Ansprüche **dadurch gekennzeichnet, daß** als Substratfragment A ein Hydroxyl-, Carboxyl-, Keto- oder Aminogruppen tragendes Substratfragment gewählt wird.

10. Wirkkomplexe nach einem der vorangehenden Ansprüche **dadurch gekennzeichnet, daß** als Substratfragment A eines der Oligopeptide Pro-Leu-Ala-Leu-Trp-Ala-Arg, Pro-Leu-Ala-Tyr-Trp-Ala-Arg, Pro-Leu-Gly-Leu-Trp-Ala-D-Arg-NH2, Pro-Leu-Gly-Cys(Me)-His-Ala-D-Arg-NH2, Pro-Tyr-Ala-Tyr-Trp-Met-Arg, Pro-Leu-Gly-Met-Trp-Ser-Arg, Pro-Leu-Gly-Leu-Trp-Ala-D-Arg-NH2, Pro-β-cyclohexyl-Ala-Gly-Cys (Me)-His-Ala-Lys (N-Methyl-Abz)-NH2, MeOSuc-Ala-Ala-Pro-Val gewählt wird.

11. Wirkkomplexe nach einem der vorangehenden Ansprüche **dadurch gekennzeichnet, daß** als Substratfragment A ein Oligosachcharid mit mindestens 2 und bis zu 20 Disaccharideinheiten gewählt wird.

12. Wirkkomplexe nach einem der vorangehenden Ansprüche **dadurch gekennzeichnet, daß** ein Substratfragment mit zwei, drei, vier oder mehr verschiedenen Wirkstoffen verknüpft ist.

13. Wirkkomplexe nach einem der vorangehenden Ansprüche **dadurch gekennzeichnet, daß** sie zusätzlich mit Alkylgruppen, besonders bevorzugt mit C12 bist C18-Alkylgruppen derivatisiert sind und insbesondere Fettsäurereste wie Palmitoyl (C16), Myristyl (C14), Lauryl (C12) oder Stearyl (C18)-Reste enthalten.

14. Verwendung von Wirkkomplexe nach einem der vorangehenden Ansprüche zur nicht therapeutischen Behandlung des Menschen.

15. Topische kosmetische Zubereitungen enthaltend eine oder mehrere Verbindungen nach einem der Ansprüche 1 bis 13.

16. Topische kosmetische Zubereitungen nach Patentanspruch 15 **dadurch gekennzeichnet, daß** Wirkstoffe B zugleich in substratgebundener und nicht substratgebundener Form enthalten sind.

17. Verwendung von Zubereitungen nach Patentanspruch 15 und 16 zur Behandlung von und zum Schutz vor altersbedingten und/oder UV-induzierten Hautveränderungen (z.B. Sonnenbrand, Altersflecken, photoaging, uneven skin tone, Pigmentierungsstörungen, Schwächung der dermal-epidermalen Junktionszohne, Reduktion der Papillen, Verminderung der mechanischen Belastbarkeit), Hautschäden oder entzündlichen Prozessen (z.B. Heliodermatits, Cuperose), Trockenheit, Rauhigkeit und Schlaffheit der Haut, Faltenbildung, der verminderten Rückfettung durch Talgdrüsen, und einer vergrößerten Anfälligkeit gegenüber mechanischem Streß (Rissigkeit), zur Behandlung von Photodermatosen, den Symptomen der senilen Xerosis und der Lichtalterung (Photoaging).

18. Verwendung von Zubereitungen nach Patentanspruch 15 und 16 zum Schutz vor altersbedingten und/oder UV-abhängigen degenerativen Erscheinungen, die mit einem Abbau des Bindegewebes der Haut verbunden sind, besonders bevorzugt dem Schutz vor Abbau von Kollagen, insbesondere von Kollagen 1, 3, 4, 5 und/oder 7; dem Schutz vor Verlust und Erhalt der Hautelastizität/elastischen Fasern; dem Erhalt bzw. die Steigerung der Hautfestigkeit (firming, slimming, contouring, cellular firming), dem Erhalt der Geschmeidigkeit und Feuchtigkeit der Haut.

19. Kosmetische Zubereitungen nach Ansprüchen 1-18 enthalten in einem beschrifteten Packmittel, dessen Beschriftung auf eine Verwendung gemäß Anspruch 17-18 hinweist.
